(19) Europäisches Patentamt
European Patent Office
Office européen des brevets

(11) Publication number: **0 404 374 A2**

(12) # EUROPEAN PATENT APPLICATION

(21) Application number: **90306004.4**

(51) Int. Cl.$^5$: **C12N 9/99**

(22) Date of filing: **01.06.90**

(30) Priority: **20.06.89 GB 8914168**
**30.11.89 GB 8927051**

(43) Date of publication of application:
**27.12.90 Bulletin 90/52**

(84) Designated Contracting States:
**AT BE CH DE DK ES FR GB GR IT LI LU NL SE**

(71) Applicant: **IMPERIAL CHEMICAL INDUSTRIES PLC**
**Imperial Chemical House, Millbank**
**London SW1P 3JF(GB)**

(72) Inventor: **Austin, Peter William**
**45 Randale Drive**
**Bury BL9 8NF(GB)**
Inventor: **Greenhalgh, Malcolm**
**Dale Cottage, Lower Park Royd Drive, Kebroyd**
**Ripponden, W.Yorkshire HX6 3HR(GB)**
Inventor: **Morpeth, Fraser Forrest**
**6 Gleneagles Way**
**Ramsbottom, Bury BL0 9QN(GB)**

(74) Representative: **James, David Gomer et al**
**Imperial Chemical Industries PLC Legal Department: Patents Po Box 6 Bessemer Road**
**Welwyn Garden City Herts, AL7 1HD(GB)**

(54) **Composition.**

(57) A medium susceptible to spoilage enzymes contains a halogen compound selected from alkali metal tetrahaloaurate, a heterocyclic compound containing at least two ring nitrogen atoms and at least one nitrogen-halogen bond, an iodonium compound and a sulphonamide containing at least one nitrogen-halogen bond. The medium may be a an aqueous solution containing soluble cellulose in which the spoilage enzyme is cellulase, for example as in an aqueous paint. the presence of the halogen compound reduces the extent of thinning of the solution. Deterioration due to lipases, proteases or amylase can also be reduced.

EP 0 404 374 A2

# COMPOSITION

The present invention relates to compositions, particularly to compositions containing spoilage enzymes and is specifically concerned with compositions in which the deleterious effects of a spoilage enzyme are reduced.

Spoilage enzymes are enzymes which cause undesired deterioration of a system due to the attack of the enzymes on a component of the system. Spoilage enzymes include, but are not restricted to, various types of cellulase, lipase, protease and amylase. Cellulase can cause breakdown of cellulose and this is particularly undesirable in systems in which a water soluble cellulose is used as a thickener in an aqueous system, for example an aqueous paint system such as an emulsion paint. The action of cellulase in such a system can result in thinning of the paint with time and this causes a deterioration in the characteristics of the paint. Lipase can cause breakdown of lipids, for example triglycerides and other emulsifying esters. The action of lipase in an emulsion containing a lipid as an emulsifying agent results in a breakdown of the lipid to acid and the alcohol (which may be a diol, triol or polyol) and this in turn can cause a deterioration in, or even the complete breakdown of, the emulsion. Protease can cause the breakdown of proteins and at least some of the breakdown products can lead to an unpleasant odour. Proteins, for example casein, can be used in adhesives and the action of protease can cause an undesirable loss in the adhesion properties. Amylase breaks down starch to mono-and di- saccharides. There are many uses for starch. Thus, starch is used in adhesives and the breakdown of the starch due to amylase can cause an undesirable loss in adhesion properties. Starch may also be used in ceramic glazes, to stabilise synthetic polymer emulsions, for the production of paper board or corrugated paper, in paper coatings and in thickeners and may be used in drilling muds. The breakdown of starch due to amylase can lead to a loss in viscosity or to a loss of integrity of a system containing the starch, such losses being undesirable and causing a deterioration in the properties of the system.

In the past, materials such as cellulose which are susceptible to enzyme deterioration have been preserved by mercuric salts. However, because of the toxic characteristics of mercuric salts, the use of these materials as preservatives is undesirable and has been banned in a number of countries. Hence, it is desirable to find an alternative to mercuric salts for use as a preservative.

A paper by Wood et al, Biochem.J. (1980), 189, pages 51 to 65, reports, inter alia, on the effect of various additives on the activity of purified components of the cellulase complex. Most of the additives tested showed little or no effect, positive or negative, on the activity of the enzyme but N-bromosuccinimide is disclosed as being a potent inhibitor. However, N-bromosuccinimide is not entirely satisfactory when used as an additive to an emulsion paint containing water soluble cellulose since it causes discolouration of the paint. Additionally, N-bromosuccinimide has an undesirable toxicity.

We have now found that other compounds which are generally less toxic than N-bromosuccinimide and in many cases give less discoloration are effective in reducing the effects due to the presence of spoilage enzymes such as cellulase, lipase, protease and amylase.

According to the present invention there is provided a medium which is susceptible to deterioration by spoilage enzymes and which contains at least one halogen compound selected from alkali metal tetrahalogenoaurates, heterocyclic compounds containing at least two ring nitrogen atoms and at least one nitrogen-halogen bond, iodonium compounds and sulphonamides containing at least one nitrogen-halogen bond.

The halogen compound is present in the medium in an amount sufficient to reduce the effect of the spoilage enzyme which is present.

The medium which is susceptible to deterioration by spoilage enzymes may be cellulose, an emulsifying ester, a protein or starch. Typically the medium is present together with one or more other materials which may act as carriers for the medium, for example a liquid which may be a solvent or dispersant for the medium or a solid on which the medium is absorbed.

A medium susceptible to deterioration is an aqueous system containing cellulose. The aqueous solution typically contains from 0.01 up to 25% by weight of cellulose, preferably dissolved cellulose, and particularly from 0.05 up to 10% by weight of dissolved cellulose. For many applications the solution contains from 0.1 up to 2% by weight of dissolved cellulose. The dissolved cellulose is particularly a cellulose ether, for example carboxymethyl cellulose, methyl hydroxyethyl cellulose, ethyl hydroxyethyl cellulose, and hydroxypropyl cellulose.

A further medium susceptible to deterioration is one containing an emulsifying ester such as an ester of a long chain carboxylic acid and glycerol, for example 1,2,3-tris[cis-9-octadecenoyl] glycerol. The ester is used in an amount sufficient to give an emulsifying effect, typically from 0.1% up to 20% by weight of the

total emulsion, the amount of ester used being dependent on the components of the emulsion.

A yet further medium susceptible to deterioration is one containing a protein such as casein, soybean protein or gelatin. The protein can be a component of an adhesive, for example in an animal glue, or can be used as a latex thickener. The proportion of protein present is dependent upon the components of the adhesive or latex and may be in the range 0.1 to 25% by weight of the total composition.

A further medium susceptible to deterioration is one containing a starch which may be, for example, wheat starch, tapioca starch, potato starch or particularly corn starch. The starch can be a component of an adhesive, a paper coating composition, a ceramic glaze, a thickener, in a polymer suspension or in other systems as described previously herein and as generally known by skilled workers. The proportion of starch present is dependent on any other components of the system and also the particular system and is typically at least 0.1% by weight and in general does not exceed 50% by weight, for example 2 to 12% by weight in paper coating and 15 to 30% in adhesives.

The at least one halogen compound is typically present in an amount of from 0.001 up to 1000 millimole per $dm^3$ of the total composition containing the medium which is susceptible to deterioration and especially is present in an amount from 0.005 up to 25 millimole per $dm^3$ of the total composition. The total composition is typically a liquid for example an aqueous solution or an emulsion.

The present invention also provides a method of treating a system which is susceptible to deterioration by spoilage enzymes, said method comprising incorporating into said system at least one halogen compound of the type specified herein. The halogen compound is incorporated into the system in an amount sufficient to reduce the effect of the at least one spoilage enzyme.

If the halogen compound is an alkali metal halogenoaurate the alkali metal can be any of the alkali metals but is typically sodium or potassium. The halogen may be any halogen but typically is bromine or chlorine. If it is desired to minimise discoloration, it is preferred that the halogen is chlorine. Sodium tetrachloroaurate is an example of an alkali metal halogenoaurate and has been found to be particularly effective. However, the alkali metal halogenoaurates are not the preferred halogen compounds on economic grounds.

The halogen compound may be a heterocyclic compound containing at least two ring nitrogen atoms and at least one nitrogen-halogen bond. There are many types of compound of this type which may be used in accordance with the present invention. Halogen compounds of this type include glycoluril derivatives, hydantoin derivatives, cyanuric acid derivatives and triazole derivatives, all containing at least one nitrogen-halogen bond.

Glycoluril derivatives which may be used are typically of the formula:

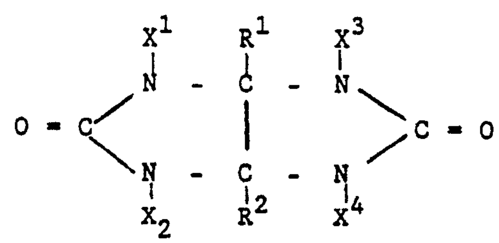

where

$X^1$, $X^2$, $X^3$ and $X^4$, which may be the same or different, are hydrogen, halogen, a hydrocarbon group, a hydrocarbonoxy group, a substituted hydrocarbon group or a substituted hydrocarbonoxy group and at least one of $X^1$, $X^2$, $X^3$ and $X^4$ is halogen;

$R^1$ and $R^2$, which may be the same or different, are hydrogen, halogen, a hydrocarbon group, a hydrocarbonoxy group, a substituted hydrocarbon group or a substituted hydrocarbonoxy group.

Preferred glycoluril derivatives are those in which $X^1$, $X^2$, $X^3$ and $X^4$ are all halogen, and in particular are all chlorine. $R^1$ and $R^2$ are typically hydrogen or a hydrocarbon group, for example an alkyl group containing up to 24 carbon atoms, and especially a lower alkyl group, that is an alkyl group containing up to 6 carbon atoms such as a methyl group. Useful glycoluril derivatives are tetrachloro glycoluril and tetrachlorodimethyl glycoluril.

Hydantoin derivatives which may be used are typically of the formula:

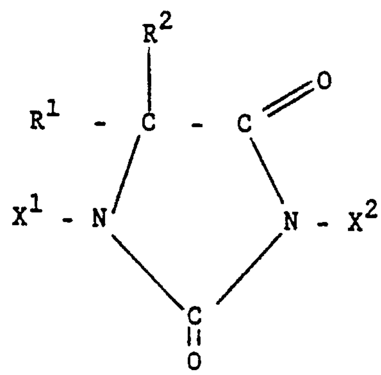

where $X^1$, $X^2$, $R^1$ and $R^2$ are as hereinbefore defined.

Preferred hydantoin derivatives are those in which both $X^1$ and $X^2$ are halogen and in particular are both chlorine. $R^1$ and $R^2$ are typically hydrogen or lower alkyl such as methyl. A useful hydantoin derivative is N,N′-dichlorodimethyl hydantoin.

Cyanuric acid derivatives which may be used are typically of the formula:-

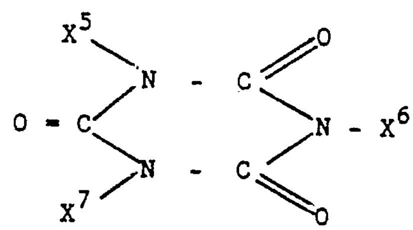

where

$X^5$, $X^6$ and $X^7$, which may be the same or different, are hydrogen, halogen, a hydrocarbon group, a hydrocarbonoxy group, a substituted hydrocarbon group, a substituted hydrocarbonoxy group or an alkali metal, and at least one of $X^5$, $X^6$ and $X^7$ is halogen.

Suitable cyanuric acid derivatives are those in which at least two of the groups $X^5$, $X^6$ and $X^7$ are halogen, conveniently chlorine, and any of $X^5$, $X^6$ and $X^7$ which is not halogen is hydrogen or an alkali metal, typically sodium. Useful cyanuric acid derivatives are dichloroisocyanuric acid and the sodium salt of dichloroisocyanuric acid.

Triazole derivatives which may be used include 1,2,3-triazoles and 1,2,4-triazoles in which there is a nitrogen-halogen bond, particularly those in which the halogen is chlorine, for example as in 1-chloro-1,2,4-triazole.

The halogen compound may be an iodonium compound and is especially a compound of the general formula:

$R^3 - I(A)_n$

where

$R^3$ is a hydrocarbon group or a substituted hydrocarbon group;

A is an anionic group having a valency of x; and

n has a positive value such that nx has a value of two.

$R^3$ is typically an aromatic group such as a benzene ring as in a phenyl iodonium compound. The group A is typically an anionic group having a single or double charge, when the value of n is, correspondingly, two or one. The anionic group may be derived from a carboxylic acid such as acetic acid, for example as in the compound phenyl iodonium di-acetate.

The halogen compound may be a sulphonamide, typically a compound of the formula:

$R^3SO_2NX^1X^2$

where $R^3$, $X^1$ and $X^2$ are all as hereinbefore defined.

The group $R^3$ is typically an aromatic group such as a benzene ring as in a benzenesulphonamide derivative. $X^1$ and $X^2$ are preferably both halogen, especially both are chlorine. A suitable sulphonamide derivative is N,N-dichlorobenzenesulphonamide.

The halogen compounds which may be used in the media of the present invention are known

compounds and can be prepared by known processes.

We have found that these compounds are effective in reducing the susceptibility of a medium to deterioration by spoilage enzymes. Specifically the halogen compounds are effective in reducing the amount of thinning of aqueous solutions containing soluble cellulose due to enzymatic activity. More specifically, in the presence of halogen compounds of the type defined, the viscosity of a solution containing soluble cellulose and the enzyme cellulase changes only slightly over a period of 60 minutes under conditions as set out in the examples. Typically, in the presence of the specified halogen compounds, the viscosity decreases under the test conditions by less than 15%, and the presence of preferred compounds results in a viscosity decrease of less than 10% and especially of less than 5%.

The halogen compounds are also effective in reducing the breakdown of emulsifying esters such as triolein (1,2,3-tris[cis-9-octadecenoyl] glycerol) in aqueous emulsions. The breakdown of triolein in the presence of the enzyme lipase is considerably reduced in the presence of a halogen compound of the type specified. The activity of the lipase may be reduced to less than 50% of its unmodified activity and preferably is reduced to less than 20% of its unmodified activity, the unmodified activity being that of the enzyme in the absence of the added halogen compound.

Additionally, the halogen compounds are effective in reducing the breakdown of proteins in an aqueous medium. In the presence of a suitable amount of a halogen compound of the type specified, the activity of the enzyme protease from different sources can be reduced to less than 30%, and very preferably to less than 10%, of its unmodified activity.

Furthermore, the halogen compounds are effective in reducing the breakdown of starch in an aqueous medium in which the starch may be dissolved or suspended and is generally partly in solution and partly in suspension. In the presence of a suitable amount of a halogen compound of the type specified, the effect of amylase in causing the breakdown of starch is considerably reduced such that little, if any, breakdown of the starch is observed. Thus, the activity of amylase can be reduced to less than 25%, preferably to less than 10%, of its unmodified activity.

Compounds giving especially useful results include sodium tetrachloroaurate; tetrachloroglycoluril; $N,N^1$-dichlorodimethyl hydantoin; 1-chloro-1,2,4-triazole; dichloroisocyanuric acid; tetrachlorodimethyl glycoluril and phenyl iodonium di-acetate.

According to a preferred aspect of the present invention there is provided an aqueous solution or emulsion of soluble cellulose, an emulsifying ester, a protein or a starch which contains either or both of $N,N^1$-dichlorodimethyl hydantoin or dichloroisocyanuric acid.

The preferred compounds used in accordance with the present invention are generally of lower toxicity than compounds previously disclosed for reducing the effects of spoilage enzymes, for example N-bromosuccinimide which is useful for reducing the thinning of aqueous cellulose solutions. Furthermore, we have found that the preferred compounds used in accordance with the present invention show a reduced tendency to discolour than prior art compounds such as N-bromosuccinimide.

The compositions of the present invention may be used as a component of an aqueous system, which may be an aqueous paint system such as an emulsion paint, or may be a cosmetic formulation or may be an adhesive composition.

Thus, as a further aspect of the present invention there is provided an aqueous paint system which contains water soluble cellulose, an emulsifying ester, or a protein and a halogen compound which is an alkali metal tetrahalogenoaurate, a heterocyclic compound containing at least two ring nitrogen atoms and at least one nitrogen-halogen bond, an iodonium compound or a sulphonamide containing at least one nitrogen-halogen bond.

The paint typically contains from 0.1 up to 25% by weight of at least one of water soluble cellulose, emulsifying ester and protein. The halogen compound is typically present in the paint in an amount of from 1 up to 1000 millimole per $dm^3$ of the paint.

In addition to water soluble cellulose, emulsifying ester and/or protein and the halogen compound, the paint also includes the usual components of an aqueous paint. Thus, the paint may additionally contain one or more of components which are a film forming component, a pigment or colorant of one sort or another, an extender,a dispersing or stabilising agent (this is usually a surfactant), a coalescing solvent, a defoamer, a rheology control agent, a wetting agent, a plasticiser, an anti-corrosive additive or an anti-freeze agent. The paint may additionally include a thickener in addition to the water-soluble cellulose and/or protein. The at least one halogen compound which is present in the composition of the present invention is an anti-microbial compound and is effective as an inhibitor of microbial cellulases and other enzymes. However, the paint may contain a further additive having anti-microbial properties and, in particular, the paint may include a fungicide, particularly one which gives protection against fungal attack to a film of the paint when applied to a surface. Materials which may be incorporated into paints are well known and are described in various

text books such as "Introduction to Paint Chemistry and Principles of Paint Technology" by G.P.A.Turner, Third Edition published 1988 by Chapman and Hall Ltd., London.

The aqueous paint system is typically an emulsion paint. Paints of this type can be prepared using techniques known in the paint industry such as milling, for example using a ball or bead mill, or mixing for example using a heavy-duty mixer or pug mixer.

Cellulose may also be used in cosmetic formulations in which it is typically present as a gelling agent. Proteins are also present in some cosmetic formulations. Breakdown of cellulose or protein due to enzymatic activity in such cosmetic formulations is undesirable and the inclusion of the specified halogen compounds in accordance with the present invention can reduce the extent of breakdown of the cellulose and/or protein.

Thus, as yet a further aspect of the present invention there is provided a cosmetic formulation which includes at least one of cellulose and protein and which also includes a halogen compound of which is an alkali metal tetrahalogenoaurate, a heterocyclic compound containing at least two ring nitrogen atoms and at least one nitrogen-halogen bond, an iodonium compound or a sulphonamide containing at least one nitrogen-halogen bond.

The cosmetic formulation can be produced by techniques known for the production of such formulations, for example by a simple mixing process, optionally at a slightly elevated temperature. The halogen compound can be added at any suitable stage, for example not later than the addition of cellulose or protein to the formulation.

Proteins and starches may also be components of adhesives, for example proteins are present in glues obtained from various animal sources and starch is commonly present in wallpaper adhesives or pastes. Enzymatic deterioration of the protein or starch in adhesive systems can result in a reduction in, or loss of, bonding strength and also, in the case of an adhesive containing protein, to unpleasant odours. Such effects may be reduced using a halogen compound of the type specified herein.

Thus, as a yet further aspect of the present invention there is provided an adhesive composition, for example a glue or a paste, which contains at least one of protein and starch and which also includes a halogen compound which is an alkali metal tetrahalogenoaurate, a heterocyclic compound containing at least two ring nitrogen atoms and at least one nitrogen-halogen bond, an iodonium compound or a sulphonamide containing at least one nitrogen-halogen bond.

Adhesives compositions can be prepared using known techniques, for example using a heavy-duty mixer.

Various aspects of the present invention are set out in more detail hereafter in the following illustrative examples in which all parts and percentages are by weight unless otherwise stated.

Examples 1 to 9

To determine cellulase activity, $15cm^3$ of a 1% w/v solution of carboxymethyl cellulose in $50mM/dm^3$ of a tris (hydroxymethyl) aminomethane/hydrochloric acid buffer having a pH of 9, was incubated at 25°C with $0.01cm^3$ of a $10mg.cm^{-3}$ aqueous solution of cellulase from Aspergillus niger obtained from the Sigma Chemical Company.

The viscosity of the cellulose solution was determined after being incubated with the cellulase for either 10 or 60 minutes

To determine the effect of an additive on the spoilage activity of the enzyme, the compound was incubated with the enzyme at 25°C for a period of one hour, the concentration of the additive being 10mM $dm^{-3}$ . The pre-incubated mixture was then added to a carboxymethyl cellulose solution of the type previously described. The resulting mixture was incubated and the viscosity was determined after 10 and 60 minutes incubation.

Details of the additive used, and the effect on the solution viscosity, are set out in Table One.

Table One

| Example | Halogen Compound (a) | Flow Rate (b) | | | |
|---|---|---|---|---|---|
| | | With additive | | Without additive | |
| | | 10 | 60 | 10 | 60 |
| 1 | STCA | 23.80 | 23.70 | 14.20 | 9.50 |
| 2 | TCG | 23.60 | 23.00 | 14.20 | 9.40 |
| 3 | DCMH | 23.10 | 22.90 | 14.40 | 9.40 |
| 4 | CT | 23.50 | 22.90 | 14.20 | 9.50 |
| 5 | DCIA | 22.10 | 22.30 | 13.00 | 9.00 |
| 6 | TCMG | 22.80 | 22.50 | 14.10 | 9.50 |
| 7 | PIDA | 22.60 | 21.80 | 14.20 | 9.40 |
| 8 | DCIS | 21.90 | 19.40 | 14.00 | 9.60 |
| 9 | DCBS | 21.20 | 18.80 | 13.80 | 9.40 |

Notes to Table One

(a) STCA is sodium tetrachloroaurate
TCG is tetrachloroglycoluril
DCMH is N,N$^1$-dichlorodimethyl hydantoin
CT is 1-chloro-1,2,4-triazole
DCIA is dichloroisocyanuric acid
TCMG is tetrachloro-dimethyl glycoluril
PIDA is phenyi iodonium di-acetate
DCIS is the sodium salt of dichlorosiocyanuric acid
DCBS is N,N-dichlorobenzene sulphonamide.
(b) 10 and 60 indicate the viscosity as measured after incubating the mixture for 10 and 60 minutes respectively.
Viscosity was measured in a Technics U-Tube viscometer (Ostwald type), size C.
The time, in minutes, for the solution to flow between the markers on the viscomenter is noted in the Table One. (In the absence of added cellulase, the time to flow between the markers is about 24 minutes).

Examples 10 and 11

N,N$^1$-dichlorodimethyl hydantoin in an amount of 9 millimoles was dissolved in ethanol and added to a lipase in a tris (hydroxymethyl) aminomethane/hydrochloric acid buffer at a pH of 9.2. The mixture was incubated for 30 minutes at 25 °C and neutralised by the addition of thiosulphate. The activity of the enzyme was determined as described hereafter.

Lipase activity was determined by the turbidimetric method described on pages 26 to 34 of "Methods of Enzymatic Analysis" Third Edition, edited by H V Bergmeyer and published by Verlag Chemie. The decrease in the light scattering of triolein (1,2,3-tris[cis-9-octadecenoyl]glycerol) by lipase was measured at 25 °C and 350 nanometres on a Perkin-Elmer Lambda 15 spectrophotometer. Light scattering was measured on 2.8cm$^3$ of an aqueous solution containing 24 micromoles of "Tris" buffer at pH 9.2, one micromole of calcium chloride, 35 micromoles of sodium chloride, 17.5 micromoles of sodium deoxycholate and 0.28 micromole of triolein. The activity was determined by the addition of 5mm$^3$ of an aqueous solution containing 4.5mg cm$^{-3}$ of lipase. The results obtained are set out in Table Two.

Table Two

| EXAMPLE | ENZYME (c) | % of activity retained (d) |
|---|---|---|
| 10 | CC | 14 |
| 11 | PS | 45 |

Notes to Table Two

(c) CC is lipase from Candida cylindracea and is available from Sigma Chemical Company, catalogue number L1754.
PS is lipase from Pseudomonas species and is available from Sigma Chemical Company, catalogue number L9518.
(d) % of activity retained is relative to a control system which was incubated in the absence of N,N[1]-dichlorodimethyl hydantoin.

Examples 12 to 15

N,N[1]-dichlorodimethyl hydantoin was dissolved in ethanol in a known amount and was added to a protease in a 0.1M potassium phosphate buffer containing potassium dihydrogen phosphate and dipotassium hydrogen phosphate at pH7. The mixture was incubated for one hour at 25°C and the enzyme activity was then compared to that of a control by monitoring the protease catalysed hydrolysis of an appropriate chromogenic substrate.

The results obtained are set out in Table Three.

Table Three

| EX | DCMH (mM) (a) | Enzyme (e) | Substrate (f) | % of activity retained (h) |
|---|---|---|---|---|
| 12 | 0.9 | C | BTEE | 66 |
| 13 | 9.0 | C | BTEE | 29 |
| 14 | 0.9 | PS | BTEE | 88 |
| 15 | 9.0 | PS | BTEE | 9 |

Notes to Table Three

(a) is as defined in Notes to Table One.
(e) C is chymotrypsin (EC 3.4.21.1).
PS is a protease from S. griseus and is available from Sigma Chemical Company, catalogue number P0652.
(f) BTEE is N-benzoyl-L-tyrosine ethylester.
(h) % of activity retained is relative to a control system which was incubated in the absence of N,N[1]-dichlorodimethyl hydantoin.

Examples 16 and 17

The effect of N,N[1]-dichlorodimethyl hydantoin in a paint formulation containing cellulose in the presence of cellulase was studied.

A millbase was prepared by stirring together the ingredients as set out in Table Four.

Table Four

| Component (i) | Amount (g) |
|---|---|
| Water | 1400 |
| 0731 | 250 |
| CMC | 40 |
| EG | 300 |
| WS | 100 |
| P208 | 20 |
| 1512M | 30 |
| DL | 100 |
| AT-1 | 425 |
| R-CR6 | 3240 |
| Notes to Table Four | |

(i) 0731 is a dispersant available from Rohm and Haas (UK) Limited as Oratan 731.
CMC is a carboxymethyl cellulose available from Aqualon (UK) Limited as Blanose CMC type 7MC.
EG is ethylene glycol.
WS is white spirit.
P208 is a wetting agent available from Lankro Chemicals as Levelan P208.
1512M is a defoamer available from Aqualon (UK) Limited as Hercules 1512M.
DL is an extender available from Steetley Minerals Limited as Dicalite.
AT-1 is talc available from Norwegian Talc (UK) Limited as Microtalc AT-1
R-CR6 is rutile titanium dioxide available from Tioxide Group Limited as Tioxide R-CR6.

575g of the millbase obtained was mixed with 341g of acrylic latex (Revacryl 1A), 2.5cm$^3$ of a gellant (Tilcom available from Till Central Laboratories, Stockton-on-Tees), and 57.5cm$^3$ of tap water and the pH adjusted to pH 9 with ammonia.

To a sample of the paint obtained was added N,N[1]-dichlorodimethyl hydantoin at a level of 100 ppm by

weight. To a further sample of paint was added the enzyme cellulase (500 ppm) from Aspergillus niger obtained from Sigma Chemical Company. To a third sample of paint were added both N,N'-dichlorodimethyl hydantoin (100 ppm) and, immediately after, cellulase (500 ppm).

The viscosity of samples of the paint was measured at various times. Viscosity was measured using a Haake Rotovisco RV2 rheometer fitted with a PKV 1° cone and plate sensor system. Viscosity was measured at sheer rate of 114.5 $sec^{-1}$ and at a temperature of 25° C.

The viscosity measurements are set out in Table Five.

Table Five

| Time (days) | Viscosity (Pas) (j) | | |
|---|---|---|---|
| | 16 | 17 | A |
| 0 | 0.8682 | 0.8088 | 0.785 |
| 0.5 | 0.8623 | 0.785 | 0.7374 |
| 1 | 0.8088 | 0.7255 | 0.5055 |
| 3 | 0.8088 | 0.7374 | 0.339 |
| 7 | 0.8444 | 0.8147 | 0.2496 |
| 14 | 0.9336 | 0.8088 | 0.2438 |
| 28 | 0.8088 | 0.666 | 0.1725 |

Notes to Table Five

(j) Viscosity (in pascal second) is determined as defined.
16 is a sample of paint containing N,N'-dichlorodimethyl hydantoin (100 ppm).
17 is a sample of paint containing N,N'-dichloromethyl hydantoin (100 ppm) and cellulase (500 ppm).
A is a sample of paint containing cellulase (500 ppm) in the absence of added N,N' dichloromethyl hydantoin.

## Example 18

A sample of amylase (1,4-α-D-glucan-glucanhydrolase; EC 3.2.1.1) from a Bacillus species (amylase sample obtained from the Sigma Chemical Company) was tested for activity in the breakdown of starch in the presence and absence of N,N'-dichlorodimethyl hydantoin.

The activity of the amylase was estimated by a modification of the method described in Methods in Enzymology, published by the Academic Press, Volume 1, on page 149.

1g of potato amylopectin (a soluble starch) was dissolved in $200cm^3$ of a 20mM sodium phosphate aqueous buffer solution adjusted to pH7. $1cm^3$ of this solution was equilibrated in a water bath maintained at 25°C. To the foregoing solution were added $2mm^3$ of an aqueous solution containing amylase in a concentration of 1mg of amylase/$cm^3$ of solution. The mixture was incubated at 25°C for five minutes. The reaction was then stopped by the addition of $1cm^3$ of a 0.4M aqueous sodium hydroxide solution containing $10mg/cm^3$ of 3,5-dinitrosalicylate and $0.3g/cm^3$ of sodium potassium tartrate. The resultant mixture was placed in a boiling water bath for five minutes, cooled rapidly and diluted with $10cm^3$ of water (deionised and subsequently doubly distilled in glass apparatus). The absorbance of the resulting mixture at 540nm was determined using a Perkin Elmer LAMBDA 15 spectrophoto meter.

When the amylase only was added to the starch, an intense colour was produced which indicated the formation of a considerable proportion of sugar due to the breakdown of the starch.

When the foregoing procedure was carried out using the amylase which had been incubated for 90

minutes with N,N[1]-dichlorodimethyl·hydantoin by the addition of the hydantoin compound to a concentration of 9 X $10^{-4}$M to $0.2cm^3$ of an aqueous solution containing the α-amylase at a concentration of $1.1mg/cm^3$, no breakdown of starch to sugar could be deteced, the intensity of the absorption at 540nm was essentially the same as that measured on a sample of the initial starch. This demonstrated an essentially complete loss in the activity of the amylase.

## Claims

1. A medium which is susceptible to deterioration by spoilage enzymes and which contains at least one halogen compound selected from alkali metal tetrahalogenoaurates, heterocyclic compounds containing at least two ring nitrogen atoms and at least one nitrogen-halogen bond, iodonium compounds and sulphonamides containing at least one nitrogen-halogen bond.

2. A medium as claimed in claim 1 which is cellulose, an emulsifying ester, a protein or a starch.

3. A medium as claimed in either claim 1 or claim 2 together with a carrier which is a liquid which is a solvent or dispersant for the medium or is a solid on which the medium is absorbed.

4. A medium as claimed in claim 3 which is an aqueous solution of a water soluble cellulose which is a methyl hydroxyethyl cellulose, carboxymethyl cellulose, ethyl hydroxyethyl cellulose or hydroxypropyl cellulose.

5. A medium as claimed in either claim 1 or claim 2 which contains an ester of a long chain carboxylic acid and glycerol, casein, soybean protein, gelatin, corn starch, wheat starch, tapioca starch or potato starch.

6. A medium as claimed in any one of claims 1 to 5 which contains from 0.001 up to 1000 millimole of the at least one halogen compound per $dm^3$ of the medium.

7. A medium as claimed in any one of claims 1 to 6 wherein the at least one halogen compound is a heterocyclic compound containing at least two ring nitrogen atoms and at least one nitrogen-halogen bond and is selected from glycoluril derivatives, hydantoin derivatives, cyanuric acid derivatives and triazole derivatives.

8. A medium as claimed in claim 7 wherein the at least one halogen compound is tetrachloroglycoluril, tetrachloro-dimethyl glycoluril, N,N′-dichlorodimethyl hydantoin, dichloroisocyanuric acid, the sodium salt of dichloroisocyanuric acid, or 1-chloro-1,2,4-triazole.

9. A medium as claimed in any one of claims 1 to 6 wherein the at least one halogen compound is phenyl iodonium di-acetate or N,N-dichlorobenzenesulphonamide.

10. A medium as claimed in any one of claims 1 to 9 which is an aqueous paint system which includes as a component thereof at least one of cellulose, an emulsifying ester or a protein.

11. A medium as claimed in any one of claims 1 to 4 which is a cosmetic formulation which includes as a component thereof a cellulose and/or a protein.

12. A medium as claimed in any one of claims 1 to 3 which is an adhesive composition which includes as a component thereof at least one of a protein or starch.

13. A method in which a system which contains cellulose, an emulsifying ester, a protein or a starch and which is susceptible to deterioration by spoilage enzymes is treated by incorporating into said system at least one halogen compound in an amount sufficient to reduce the effect of any spoilage enzyme which is present wherein the said halogen compound is at least one compound selected from alkali metal tetrahalogenoaurates, heterocyclic compounds containing at least two ring nitrogen atoms and at least one nitrogen-halogen bond, iodonium compounds and sulphonamides containing at least one nitrogen-halogen bond.